(19) **Europäisches Patentamt**
**European Patent Office**
**Office européen des brevets**

(11) **EP 1 290 222 B1**

(12) **EUROPEAN PATENT SPECIFICATION**

(45) Date of publication and mention
of the grant of the patent:
**08.08.2007 Bulletin 2007/32**

(51) Int Cl.:
*C12Q 1/68* (2006.01)    *C12Q 1/70* (2006.01)
*C07H 21/02* (2006.01)    *C07H 21/04* (2006.01)
*C12P 15/00* (2006.01)    *C12P 21/02* (2006.01)
*C12P 21/06* (2006.01)

(21) Application number: **01935701.1**

(22) Date of filing: **18.05.2001**

(86) International application number:
**PCT/US2001/016210**

(87) International publication number:
**WO 2001/090414 (29.11.2001 Gazette 2001/48)**

(54) **IN VITRO EVOLUTION OF NUCLEIC ACIDS AND ENCODED POLYPEPTIDE**

IN VITRO EVOLUTION VON NUKLEINSÄUREN UND KODIERTEN POLYPEPTIDEN

EVOLUTION IN VITRO D'ACIDES NUCLEIQUES ET DE POLYPEPTIDES CODES

(84) Designated Contracting States:
**AT BE CH CY DE DK ES FI FR GB GR IE IT LI LU MC NL PT SE TR**

(30) Priority: **19.05.2000 US 206016 P**

(43) Date of publication of application:
**12.03.2003 Bulletin 2003/11**

(73) Proprietor: **Williams, Richard B.**
**South Pasadena, CA 91030 (US)**

(72) Inventor: **Williams, Richard B.**
**South Pasadena, CA 91030 (US)**

(74) Representative: **Becker Kurig Straus**
**Patentanwälte**
**Bavariastrasse 7**
**80336 München (DE)**

(56) References cited:
WO-A-98/31700       WO-A-99/51773
WO-A1-92/02536      US-A- 4 599 303
US-A- 5 462 733     US-A- 5 843 701
US-B1- 6 207 446    US-B1- 6 214 553

• SPRINZL M ET AL: "The -C-C-A end of tRNA and its role in protein biosynthesis." 1979, PROGRESS IN NUCLEIC ACID RESEARCH AND MOLECULAR BIOLOGY. 1979, VOL. 22, PAGE(S) 1 - 69 , XP008053303 ISSN: 0079-6603 * the whole document *

• FRASER T H ET AL: "SYNTHESIS AND AMINOACYLATION OF 3 AMINO-3 DEOXY TRANSFER RNA AND ITS ACTIVITY IN RIBOSOMAL PROTEIN SYNTHESIS" 1973, PROCEEDINGS OF THE NATIONAL ACADEMY OF SCIENCES OF THE UNITED STATES OF AMERICA, VOL. 70, NR. 9, PAGE(S) 2671-2675 , XP002347268 ISSN: 0027-8424 * the whole document *

• CIMINO G D ET AL: "PSORALENS AS PHOTOACTIVE PROBES OF NUCLEIC ACID STRUCTURE AND FUNCTION: ORGANIC CHEMISTRY PHOTOCHEMISTRY, AND BIOCHEMISTRY" ANNUAL REVIEW OF BIOCHEMISTRY, PALTO ALTO, CA, US, vol. 54, 1985, pages 1151-1193, XP000675262 ISSN: 0066-4154

• GASPARRO F P ET AL: "THE EXCITATION OF 8-METHOXYPSORALEN WITH VISIBLE LIGHT: REVERSED PHASE PHLC QUANTITATION OF MONOADDUCTS AND CROSS-LINKS" PHOTOCHEMISTRY AND PHOTOBIOLOGY, OXFORD, GB, vol. 57, no. 6, 1993, pages 1007-1010, XP000986300 ISSN: 0031-8655

• FAVRE ALAIN ET AL: "Thionucleobases as intrinsic photoaffinity probes of nucleic acid structure and nucleic acid-protein interactions" February 1998 (1998-02), JOURNAL OF PHOTOCHEMISTRY AND PHOTOBIOLOGY B BIOLOGY, VOL. 42, NR. 2, PAGE(S) 109-124 , XP002347269 ISSN: 1011-1344 * the whole document *

• MILDER S J ET AL: "SPECTROSCOPY AND PHOTOCHEMISTRY OF THIOURACILS IMPLICATIONS FOR THE MECHANISM OF PHOTOCROSSLINKING IN TRANSFER RNA" 1985, JOURNAL OF THE AMERICAN CHEMICAL SOCIETY, VOL. 107, NR. 25, PAGE(S) 7365-7373 , XP008053292 ISSN: 0002-7863 * the whole document *

• MATTHEAKIS ET AL.: 'An in vitro polysome display system for identifying ligands from very large peptide libraries' PROCEEDINGS OF THE NATIONAL ACADEMY OF SCIENCES vol. 91, no. 19, September 1994, pages 9022 - 9026, XP002043737

## Description

### Background of the Invention

Field of the Invention

The present invention relates to compositions and methods for the selection of nucleic acids and polypeptides.

Description of the Related Art

Ligand-receptor interactions are of interest for many reasons, from elucidating basic biological site recognition mechanisms to drug screening and rational drug design. It has been possible for many years to drive in vitro evolution of nucleic acids by selecting molecules out of large populations that preferentially bind to a selected target, then amplifying and mutating them for subsequent re-selection (Tuerk and Gold, Science 249:505 (1990)).

The ability to perform the selection process with proteins would be extremely useful. This would permit in vitro design and production of proteins that bind specifically to chosen ligands. The use of proteins, as compared to nucleic acids, is particularly advantageous because the twenty diverse amino acid side chains in proteins have far more binding possibilities than the four similar chains in nucleic acid side. Further, many biologically and medically relevant ligands bind proteins.

Both nucleic acid and protein evolution methods require access to a large and highly varied population of test molecules, a way to select members of the population that exhibit the desired properties, and the ability to reproduce the selected molecules with mutated variations to obtain another large population for subsequent selection.

Prior attempts to develop a protein evolution method were primarily limited by the inability of the proteins to reproduce themselves and the inability to link a polypeptide's encoding mRNA with the translated product. Additionally, the generation of large peptide libraries and screening methods have, until recently, required that the process have an in vivo expression step. Examples include yeast two- or three-hybrid, yeast display and phage display methods (Fields and Song, Nature 340:245 (1989); Licitra and Liu, PNAS 93:12817 (1996); Boder and Wittrup, Nat Biotechnol 15:553 (1997); and Scott and Smith, Science 249:386 (1990)). In vivo methods suffer from various disadvantages, including a limited library size and cumbersome screening steps. Additionally, undesired selective pressures can be placed on the generation of variants by cellular constraints of the host.

In vitro methods have been developed more recently, using prokaryotic and eukaryotic in vitro translation systems, such as ribosome display (Mattheakis et al., PNAS 91:9022 (1994); Hanes and Plückthun, PNAS 94:4937 (1997); Jermutus et al., Current Opinion in Biotechnology 9:534 (1998)). These methods link the protein and its encoding mRNA with the ribosome and the entire complex is screened against a ligand of choice. Potential disadvantages of this method include the large size of the ribosome, which could interfere with the screening of the attached, and relatively tiny, protein.

In 1997, two groups of workers developed an in vitro method of attaching a protein to its coding sequence during translation by using the ribosomal peptidyl transferase with puromycin attached to a linker DNA (Szostak et al., International Patent Publication WO 98/31700; Roberts and Szostak PNAS 94:12297 (1997); Nemoto et al., FEBS Letters 414:405 (1997)). Once the coding sequence and peptides are linked, the peptides are exposed to a selected ligand. Selection or binding of the peptide by the ligand also selects the attached coding sequence, which can then be reproduced by standard means. Both Roberts and Szostak and Nemoto et al. used the technique of attaching a puromycin molecule to the 3' end of a coding sequence by a DNA linker or other non-translatable chain. Puromycin is a tRNA acceptor stem analog which accepts the nascent peptide chain under the action of the ribosomal peptidyl transferase and binds it stably and irreversibly, thereby halting translation. These methods suffer from certain disadvantages. For example, the coding sequence encoding each peptide must be known and be modified both initially and between each selection. Thus, the methods of Roberts and Nemoto cannot be used to select native unknown mRNAs. Further, the modification of the coding sequence adds several steps to the process. Finally, the attached puromycin on the linker molecules may compete in the translation reaction with the native tRNAs for the A site on the ribosome reading its coding sequence or a nearby ribosome, and could thus "poison" the translation process, just as would unattached puromycin in the translation reaction solution. Inadvertent interactions between puromycin and ribosomes could result in two kinds of reaction non-specificity: prematurely shortened proteins and proteins attached to the wrong message. There are reports in the prior art that indicate that the avidity of the A site and the peptidyl transferase for the puromycin may be modulated by $Mg^{++}$ concentration (Roberts, Curr. Opin. Chem. Biol. 3:268 (1999)).

Although $Mg^{++}$ concentration may be titrated to control for the first kind of non-specificity, i.e. premature termination of translation, it will not affect the second type, i.e. inaccurate mRNA-protein linkage.

Thus, a need exists for an in vitro nucleic acid-based protein evolution system which does not necessarily require initial knowledge of the nucleic acid's sequence or repeated chemical modification of the nucleic acids, and which can accurately link a mRNA to its protein.

Summary of the Invention

The present invention provides compositions and methods to select and evolve desired properties of proteins and nucleic acids. In various embodiments, the current invention provides modified tRNA's and tRNA analogs. Other embodiments include methods for generating polypeptides, assays enabling selection of individual members of the population of polypeptides having desired characteristics, methods for amplifying the nucleic acids encoding such selected polypeptides, and methods for generating new variants to screen for enhanced properties.

In several embodiments, the present invention permits the attachment of a protein to its message without requiring modification of native mRNA, although modified mRNA may still be used. The specificity of the methods embodied in various aspects of the current invention are determined by the specificity of the codon-anticodon interaction.

In a preferred embodiment, the invention permits the selection of nucleic acids by selecting the proteins for which they code. This may be accomplished by connecting the protein to its cognate mRNA at the end of translation, which in turn is done by connecting both the protein and mRNA to a tRNA or tRNA analog.

A preferred embodiment of the invention includes a tRNA molecule capable of covalently linking a nucleic acid encoding a polypeptide and the polypeptide to the tRNA, wherein the linkage of the nucleic acid occurs on a portion of the tRNA other than the linkage of the polypeptide, the tRNA comprising a linking molecule associated with the anticodon of the tRNA. Preferably, an amino acid or amino acid analog is attached to the 3' end of a tRNA molecule by a stable bond to generate a stable aminoacyl tRNA analog (SATA).

Other embodiments include a mRNA comprising a psoralen, preferably located in the 3' region of the reading frame, more preferably at the most 3' codon of the reading frame, most preferably at the 3' stop codon of the reading frame. In preferred embodiments, linkage between the tRNA and the mRNA is a cross-linked psoralen molecule, more preferably a furan-sided psoralen monoadduct.

Several embodiments of the present invention include a method of stably linking a nucleic acid, a tRNA, and a polypeptide encoded by the mRNA together to form a linked mRNA-polypeptide complex. In a preferred embodiment, the nucleic acid is an mRNA. The method can further comprise providing a plurality of distinct nucleic acid-polypeptide complexes, providing a ligand with a desired binding characteristic, contacting the complexes with the ligand, removing unbound complexes, and recovering complexes bound to the ligand.

Several methods of the current invention involve the evolution of nucleic acid molecules and/or proteins. In one embodiment, this invention comprises amplifying the nucleic acid component of the recovered complexes and introducing variation to the sequence of the nucleic acids. In other embodiments, the method further comprises translating polypeptides from the amplified and varied nucleic acids, linking them together using tRNA, and contacting them with the ligand to select another new population of bound complexes. Several embodiments of the present invention use selected protein-mRNA complexes in a process of in vitro evolution, especially the iterative process in which the selected mRNA is reproduced with variation, translated and again connected to cognate protein for selection.

Brief Description of the Drawings

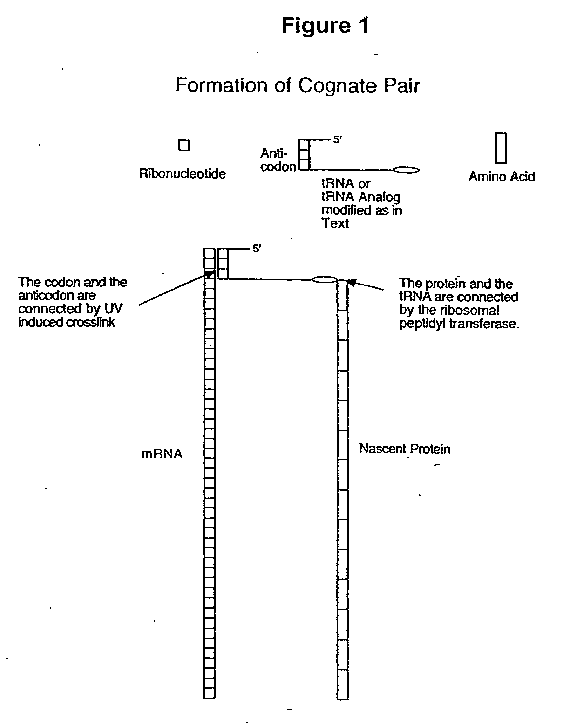

Figure 1 illustrates schematically one example of the complex formed by the mRNA and its protein product when linked by a modified tRNA or analog. As shown, a codon of the mRNA pairs with the anticodon of a modified tRNA and is covalently crosslinked to a psoralen monoadduct by UV irradiation. The translated polypeptide is linked to the modified tRNA via the ribosomal peptidyl transferase. Both linkages occur while the mRNA and nascent protein are held in place by the ribosome.

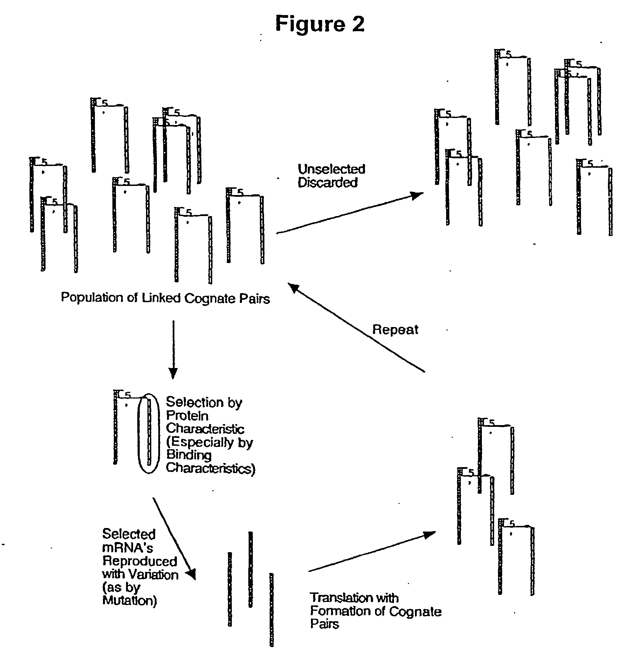

Figure 2 illustrates schematically an example of the in vitro selection and evolution process, wherein the starting nucleic acids and their protein products are linked (e.g., according to Figure 1) and are selected by a particular characteristic exhibited by the protein. Proteins not exhibiting the particular characteristic are discarded and those having the characteristic are amplified with variation, preferably via amplification with variation of the mRNA, to form a new population. In various embodiments, nonbinding proteins will be selected. The new population is translated and linked via a modified tRNA or analog, and the selection process is repeated. As many selection and amplification/mutation rounds as desired can be performed to optimize the protein product.

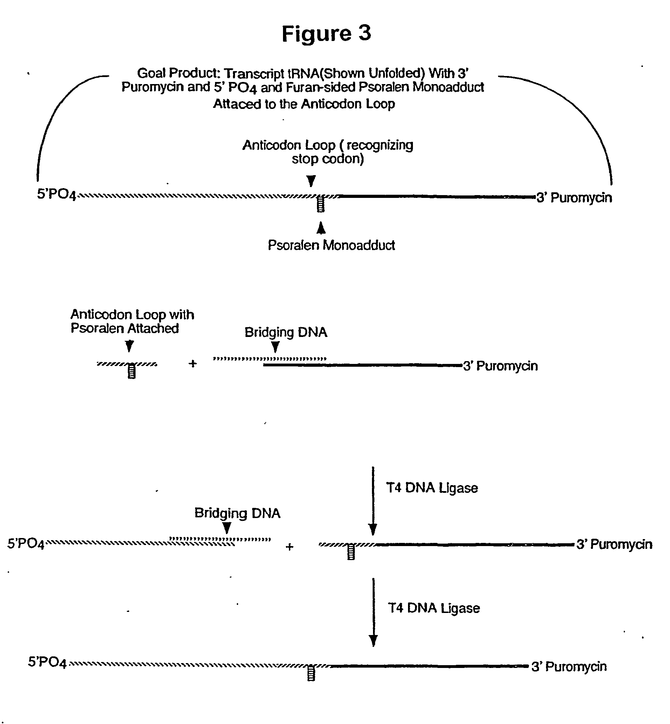

Figure 3 illustrates one method of construction of a tRNA molecule of the invention. In this embodiment, the 5' end of a tRNA, a nucleic acid encoding an anticodon loop and having a molecule capable of stably linking to mRNA (here, psoralen), and the 3' end of tRNA modified with a terminal puromycin molecule are ligated to form a complete modified tRNA for use in the in vitro evolution methods of the invention.

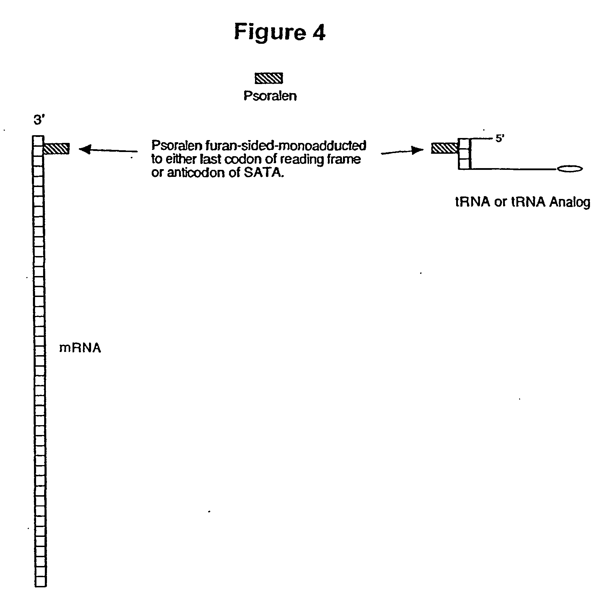

Figure 4 describes two alternative embodiments by which the crosslinking molecule psoralen can be positioned to be capable of linking the mRNA with the tRNA in the methods of the invention. A first embodiment includes linking the psoralen monoadduct to the mRNA, and a second embodiment includes linking the psoralen to the anticodon of the tRNA. Psoralen can either be monoadducted to the anticodon or the 3' terminal codon of the reading frame for known or partially known messages. This can be done in a separate procedure from translation, i.e. before translation occurs.

Detailed Description of the Preferred Embodiment

Various aspects of the present invention use a tRNA mechanism that links messenger RNA (mRNA) to its translated protein product, forming a "cognate pair." In several embodiments, mRNAs whose sequence is not known can be expressed, its protein characterized through a selection process against a ligand with desired or selected properties, and nucleic acid evolution-resulting in protein evolution-can be performed in vitro to arrive at molecules with enhanced properties. The cognate pairs are preferably attached via a linking tRNA, modified tRNA, or tRNA analog. In a preferred embodiment, the tRNA is connected to the nascent peptide by the ribosomal peptidyl transferase and to the mRNA through an ultraviolet induced cross link between the anticodon of the tRNA or tRNA analog and the codon of the RNA message. This can be done by, for example thiouracil, but in a preferred method, the linker is a psoralen crosslink made from a psoralen monoadduct pre-placed on either the mRNA or preferably on the tRNA anticodon of choice. Preferably, a tRNA stop anticodon is selected. A stop codon/anticodon pair selects for full length transcripts. One skilled in the art will understand that a mRNA not having a stop codon may also be used and that any codon or nucleic acid triplet may be used. A tRNA having an anticodon which is not naturally occurring can be synthesized according to methods known in the art (e.g. Figure 3).

The terms "protein," "peptide," and "polypeptide" are defined herein to mean a polymeric molecule of two or more units comprised of amino acids in any form (e.g., D- or L- amino acids, synthetic or modified amino acids capable of polymerizing via peptide bonds, etc.), and these terms may be used interchangeably herein.

The term "pseudo stop codon" is defined herein to mean a codon which, while not naturally a nonsense codon, prevents a message from being further translated. A pseudo stop codon may be created by using a "stable aminoacyl tRNA analog" or SATA, as described below. In this manner, a pseudo stop codon is a codon which is recognized by and binds to a SATA. Another method by which to create a pseudo stop codon is to create an artificial system in which the necessary tRNA having an anticodon complementary to the pseudocodon is substantially depleted. Accordingly, translation will stop when the absent tRNA is required, i.e. at the pseudo stop codon. One skilled in the art will appreciate that are numerous ways to create a pseudo stop codon as defined herein.

The formation of connections between mRNA and its protein product generally requires a tRNA or tRNA analog with certain characteristics. In several embodiments of the current invention, the tRNA or tRNA analog will have a stable peptide acceptor. This modification changes the tRNA or tRNA analog such that after it accepts the nascent peptide chain by the action of the ribosomal peptidyl transferase, it holds the chain in a stable manner such that the peptidyl transferase cannot detach it. This may be accomplished by using a bond such as a 2' ester on a 3' deoxy adenosine or an amino "acyl tRNA$_{ox-red}$" which can bind to the ribosome, accept the peptide chain, and then not act as a donor in the next transpeptidation (Chinali et al., Biochem. 13:3001 (1974); Krayevsky and Kukhanova, Prog. Nuc. Acid Res 23:1 (1979) and Sprinzl and Cramer Prog. Nuc. Acid Res 22:1 (1979)).

In a preferred embodiment, an amino acid or amino acid analog is attached to the 3' end of the tRNA or tRNA analog by a stable bond. This stable bond contrasts the labile, high energy ester bond that connects these two in the native structure. The stable bond not only protects the bond from the action of the peptidyl transferase, but also preserves the structure during subsequent steps. For convenience, this modified tRNA or tRNA analog will be referred to as a "stable aminoacyl tRNA analog" or SATA. As used herein, a SATA is an entity which can recognize a selected codon such that it can accept a peptide chain by the action of the ribosomal peptidyl transferase when the cognate codon is in the reading position of the ribosome. The peptide chain will be bound in such a way that the peptide is bound stably and cannot be unattached by the peptidyl transferase. Preferably, the selected codon is recognized by hydrogen bonding.

One method for creating a SATA was published in 1973 (Fraser and Rich, PNAS 70:2671 (1973)). This method involves the conversion of a tRNA, or tRNA analog, to a 3'-amino-3'-deoxy tRNA. This is accomplished by adding a 3'-amino-3'-deoxy adenosine to the end of a native tRNA with tRNA nucleotidyl transferase after removing the native adenosine from it with snake venom phosphodiesterase. This modified tRNA is then charged with an amino acid by the respective aminoacyl tRNA synthetase (aaRS). Fraser and Rich used an aaRS in which the tRNA is charged on the 3', rather than the 2', hydroxyl. The amino acid is bound to the tRNA by a stable amide bond rather than the usual labile high-energy ester bond. Thus, when it accepts a peptide from ribosomal peptidyl transferase it will stably hold the peptide and not be able to donate it to another acceptor.

In a preferred method, the SATA will be attached to the translated message by a psoralen cross link between the codon and anticodon. Psoralen cross links are preferentially made between sequences that contain complementary 5' pyrimidine-purine 3' sequences especially UA or TA sequences (Cimino et al., Ann. Rev. Biochem. 54:1151 (1985)).

The codon coding for the SATA, or the linking codon, can be PYR-PUR-X or X-PYR-PUR, so that several codons may be used for the linking codon. Conveniently, the stop or nonsense codons have this configuration. Using a codon that codes for an amino acid may require minor adjustments to the genetic code, which could complicate some applications. Therefore, in a preferred embodiment, a stop codon is used as the linking codon and the SATA functions as a nonsense suppressor in that it recognizes the linking codon. One skilled in the art, however, will appreciate that, with appropriate adjustments to the system, any codon can be used.

Fraser and Rich did their work in E. coli, but the most effective in vitro translation systems are in eukaryotes The use of prokaryotic suppressors in eukaryotic translation systems appears to be feasible (Geller and Rich Nature 283:41 (1980); Edwards et al PNAS 88:1153 (1991); Hou and Schimmel Biochem 28:6800 (1989)). They are primarily limited by the resident aaRS's. This limitation is overcome by various embodiments of the present invention because the tRNA or analog can be charged in the prokaryotic system and then purified according to established methods (Lucas-Lenard and Haenni, PNAS 63:93 (1969)).

In several embodiments of the current invention, acceptor stem modifications suitable for use in the tRNAs and analogs can be produced by various methods known in the art. Such methods are found in, for example, Sprinzl and Cramer, Prog. Nuc. Acid Res. 22:1 (1979). In an alternative embodiment, "transcriptional tRNA", i.e. the sequence of the tRNA as it would be transcribed rather than after the post-transcriptional processing, leads to the atypical and modified bases that are common in tRNAs. These transcriptional tRNAs are capable of functioning as tRNAs (Dabrowski et al., EMBO J. 14: 4872, 1995; and Harrington et al., Biochem. 32: 7617, 1993). Transcriptional tRNA can be produced by transcription or can be made by connecting commercial RNA sequences (such as those available from Dharmacon Research Inc., Boulder, CO) together, piece-wise as in Figure 3, or in some combination of established methods. For instance, the 5' phosphate and 3' puromycin are commercially available attached to oligoribonucleotides. These pieces can be connected together using T4 DNA ligase, as is well-known in the art (Moore and Sharp, Science 256: 992, 1992). Alternatively, in a preferred embodiment, T4 RNA ligase is used (Romaniuk and Uhlenbeck, Methods in Enzymology 100:52 (1983)).

In several embodiments of the present invention, psoralen is monoadducted to the SATA by construction of a tRNA from pieces including a psoralen linked oligonucleotide (Fig. 3) or by monoaduction to a native or modified tRNA or analog (Fig. 4).

In several embodiments, translation will stop when the nascent protein is attached to the SATA by the peptidyl transferase. When a large number of ribosomes are in this position the SATA and the mRNA will be connected with UV light. In a preferred method this will be accomplished by having a psoralen crosslink formed. Psoralens have a furan side and a pyrone side, and they readily intercalate between complementary base pairs in double stranded DNA, RNA, and DNA-RNA hybrids (Cimino et al., Ann. Rev. Biochem. 54:1151 (1985)). Upon irradiation with UV, preferably in the range of 320 nm to 400 nm, cross linking will take place and leave the staggered pyrimidines covalently bound. By either forming crosslinks and photo reversing them or by using selected wavelengths, it is possible to form monoadducts, described more fully below. These will be either pyrone sided or furan sided monoadducts. Upon further irradiation, the furan sided monoadducts can be covalently crosslinked to complementary base pairs. The pyrone sided monoadducts cannot be further crosslinked. The formation of the furan sided psoralen monoadduct (MAf) is also done according to established methods. In a preferred method, the psoralen is attached to the anticodon of the SATA. However, psoralen can also be attached at the end of the reading frame of the message, as depicted in Figure 4.

Methods for large scale production of purified MAf on oligonucleotides are described in the literature (e.g., Speilmann et al., PNAS 89:4514, 1992), as are methods that require less resources, but have some non-cross-linkable pyrone sided psoralen monoadduct contamination (e.g., U.S. Patent No. 4,599,303; Gamper et al., J. Mol. Biol. 197: 349 (1987); Gamper et al., Photochem. Photobiol. 40:29 (1984)). In several embodiments of the current invention, psoralen labeling is accomplished by using either method. In a preferred embodiment, furan sided monoadducts will be created using visible light, preferably in the range of approximately 400 nm - 420 nm, according to the methods described in U.S. Patent No. 5,462,733 and Gasparro et al., Photochem. Photobiol. 57:1007 (1993). In one aspect of this invention, a SATA with a furan sided monoadduct or monoadducted oligonucleotides for placement on the 3' end of mRNAs, along with a nonadducted SATA are provided as the basis of a kit.

Use of the SATA and the monoadduct in several embodiments of the current invention is particularly advantageous for in vitro translation systems. However, one skilled in the art will appreciate that in situ systems can also be used. Various embodiments of the current invention will be applicable to any in vitro translation system, including, but not limited to, rabbit reticulocyte lysate (RLL), wheat germ, E. coli, yeast lysate systems, etc. Many embodiments of the current invention are also well-suited for use in hybrid systems where components of different systems are combined.

tRNAs aminoacylated on a 3' amide bond are reported not to combine with the elongation factor EF-TU which assists in binding to the A site (Sprinzl and Cramer, Prog. Nuc. Acid Res. 22:1 (1979)). Such modified tRNAs do, however, bind to the A site. This binding of 3' modified tRNAs can be increased by changing the Mg$^{++}$ concentration (Chinali et al., Biochem. 13:3001 (1974)). The appropriate concentrations of and/or molar ratios of SATA and Mg$^{++}$ can be determined empirically. If the concentration or A site avidity of SATA is too high, the SATA could compete with native tRNAs for non-cognate codons i.e., could function much like puromycin and stall translation. If the concentration or A site avidity of SATA is too low, the SATA might not effectively compete with the release factors, i.e., it would not act as an effective nonsense suppressor tRNA. The balance between these can be determined empirically.

It is also believed that the elongation factor aids in proofreading the codon-anticodon recognition. The error rate in the absence of elongation factor and the associated GTP hydrolysis is estimated to be 1 in 100 for codons one nucleotide away (Voet and Voet, Biochemistry 2nd ed. pp. 1000-1002 (1995), John Wiley and Sons). In a preferred embodiment, UAA is used as the linking codon. For UAA as the linking codon, there are 7 non stop codons which differ by one amino acid. This is 7/61 or about 11.5% of the non stop codons. One can estimate the probability of miscoding a given codon as $(0.01)(0.115) = 1.15 \times 10^{-3}$ miscodes per codon. Thus, one would expect a miscode about every 870 codons, a frequency which will not substantially impair performance of various methods of the current invention. In an alternative embodiment, UAG is used as the linking codon.

In several embodiments, appropriate concentrations of SATA and $Mg^{++}$ are used in the in vitro translation system, e.g. RRL, in the presence of the mRNA molecules in the pool, causing translation to cease when the ribosome reaches the codon which permits the SATA to accept the peptide chain (the linking codon described above). Within a short time, most of the linking codons will be occupied by SATAs within ribosomes. In a preferred embodiment, the system then will be irradiated with UV light, preferably at approximately 320 nm to 400 nm. Nucleic acids are typically transparent to, i.e. do not absorb, this wavelength range. Upon irradiation, the psoralen monoadduct will convert to a crosslink connecting the anticodon and the codon by a stable covalent bond.

In a preferred embodiment, the target mRNA is pre-selected. In another embodiment, the target mRNA is artificially produced. In an alternative embodiment, the target consists of messages native to the system under investigation, which may be unknown and/or unidentified. The ability to use unknown and/or unidentified mRNAs is a particular advantage of several embodiments of the current invention.

In several embodiments, once all the nascent proteins are connected to their cognate mRNAs, the ribosomes are released or denatured. Preferably, this is accomplished by the depletion of $Mg^{++}$ through dialysis, simple dilution, or chelation. One skilled in the art will understand that other methods, including, but not limited to, denaturation by changing the ionic strength, the pH, or the solvent system can also be used.

In several embodiments of the invention, the selection of cognate pairs will be based upon affinity binding of proteins according to any of a variety of established methods, including, but not limited to, affinity columns, immunoprecipitation, and many high throughput screening procedures. A variety of ligands may also be used, including, but not limited to, proteins, nucleic acids, chemical compounds, polymers and metals. In addition, cell membranes or receptors, or even entire cells may be used to bind the cognate pairs. The selection can be positive or negative. That is, the selected cognate pairs can be those that do bind well to a ligand or those that do not. For instance, for a protein to accelerate a thermodynamically favorable reaction, i.e., act as an enzyme for that reaction, it should bind both the substrate and a transition state analog. However, the transition state analog should be bound much more tightly than the substrate. This is described by the equation

$$\frac{k_{enzyme}}{k_{\varphi enzyme}} = \frac{K_{trans}}{K_{subst}}$$

where the ratio of the rate of the reaction with the enzyme, $k_{enzyme}$, to the rate without, $k_{\varphi enzyme}$, is equal to the ratio of the binding of the transition state to the enzyme $K_{trans}$ over the binding of the substrate to the enzyme $K_{subst}$ (Voet and Voet, Biochemistry 2nd ed. p.380, (1995), John Wiley and Sons).

In a preferred embodiment, proteins which compete poorly for binding to the substrate but compete well for binding to the transition state analog are selected. Operationally, this may be accomplished by taking the proteins that are easily eluted from a matrix with substrate or substrate analog bound to it and are the most difficult to remove from matrix with transition state analog bound to it. By sequentially repeating this selection and reproducing the proteins through replication and translation of the nucleic acid of the cognate pairs, an improved enzyme should evolve. Affinity to one entity and lack of affinity to another in the same selection process is used in several embodiments of the current invention. Selection can also be done by RNA in many embodiments.

Once the selection has identified a population of cognate pairs it may be convenient to detach the mRNA strand from the SATA to reproduce it. This is not always necessary but can be accomplished by irradiating the pairs with UV, preferably at approximately 313 nm or just below. This has been identified as a wave length that will photoreverse the psoralen crosslink to MAf and damage the nucleic acid minimally. The ratio of photoreversal to nucleic acid damage is estimated to be 1 photoreversal for damage to 1 in 600 bases (Cimino et al., Biochem 25:3013 (1986)).

One skilled in the art will appreciate that the mRNAs can be reproduced in many ways, including, but not limited to, by RNA-dependent RNA polymerases or by reverse transcription and PCR. This can take place using mRNAs separated from the cognate pairs, e.g., using poly T or poly U to hybridize to the poly A tails of, for instance, native unknown messages or by leaving the cognate pairs intact and using oligonucleotide primers that hybridize partially into the reading frame for known messages. Alternatively, commercial kits for rapid amplification of cDNA ends may be used. When this is used to evolve proteins and not just to select them, it would be preferable to sample at least one amino acid substitution at each position in the protein.

*The Replication Threshold*

A nominal minimum number of replications for efficient evolution may be estimated using the following formulae. If there is a sequence which is *n* sequences in length, with a selective improvement *r* mutations away with a mutation rate of *p,* the probability of generating the selective improvement on replication may be determined as follows:

For r =1, probability of a mutation at the right point, *p,* times the probability that it mutated to the right one of the three nucleotides that are different from the starting point, *1/3,* times the probability that the other n-1 sites remain unmutated, *(1-p)(n-r),* or

$$P_r = \left(\frac{p}{3}\right)^1 (1-p)^{(n-1)}$$

where, P = the probability of attaining a given change r mutations away. More generally, for all r values:

$$P_r = \left(\frac{p}{3}\right)^r (1-p)^{(n-r)} \qquad .$$

It is instructive to compare the chances of finding an advantage one mutation away with the chances three mutations away. This is because, given the triplet genetic code, any given codon can only change into nine other codons in one mutation. Indeed, it turns out that no codon can actually change into nine other amino acid codes in one mutation. The maximum number of amino acids that can be accessed in one mutation is seven amino acids and there are only eight codons of the sixty-four that can do this. Most codons have five or six out of nineteen other amino acids within one mutation. To reach all nineteen amino acids that are different from the starting one requires, in general, three mutations. These three mutations cannot be sequential since the two intervening ones will not, in general, be selectively advantageous. Therefore we need to use steps that are, at least, three mutations in size (*r=3*) to use all 20 amino acids.

For a mutation rate of .0067, which is that reported for "error-prone PCR", using a message of 300 nucleotides, which gives a short protein of 100 amino acids:

$$P_3 = 1.51 \times 10^{-9}$$

Therefore, one would expect to need a threshold of :

$$\frac{1}{1.51 \times 10^{-9}} = 6.64 \times 10^8$$

replications at that mutation rate to reasonably expect to reach the next amino acid that is advantageous. This is not the replication to use since the binomial expansion shows that over 1/3 of trials (actually about 1/e) would not contain the given sequence with selective advantage.

A poisson approximation for large *n* and small *p* for a given μ can be calculated so that we can compute the general term when *n* is, say, of the order $10^9$ and *p* is of the order $10^{-9}$. The general term of the approximation is:

$$\frac{\mu^r}{r!e^\mu}$$

An amplification factor of greater than approximately 6/P ensures that evolution will progress with the use of all amino acids. This is useful when the production of novel proteins precludes the use of "shuffling" of preexisting proteins.

*Limits on Purification*

Given a reversible binding where B and C compete for A:

$$AB \leftrightarrow A + B \qquad\qquad AC \leftrightarrow A + C$$

$$k_B = \frac{[A][B]}{[AB]} \qquad k_C = \frac{[A][C]}{[AC]}$$

$$[B] = k_B \frac{[AB]}{[A]}$$
(1)

$$[C] = k_C \frac{[AC]}{[A]}$$
(2)

The total concentrations can be expressed:

$$[B]_T = [B] + [AB]$$

(3)

$$[C]_T = [C] + [AC]$$

(4)

Dividing (3) by (4)

$$[B]_T = [B] + [AB]$$
$$[C]_T = [C] + [AC]$$

And substituting (1) and (2) for [B] and [C]:

$$[B]_T = k_B\left[\frac{AB}{A}\right] + [AB]$$
$$[C]_T = k_C\left[\frac{AC}{A}\right] + [AC]$$

Rearranging:

$$\frac{[B]_T}{[C]_T} = \frac{[AB]\left(\dfrac{k_B + [A]}{[A]}\right)}{[AC]\left(\dfrac{k_C + [A]}{[A]}\right)}$$

Canceling the [A]'s in the numerator and denominator:

$$\frac{[B]_T}{[C]_T} = \frac{[AB](k_B + [A])}{[AC](k_c + [A])}$$

Finally rearranging:

$$\frac{[AB]}{[AC]} = \frac{[B]_T(k_C + [A])}{[C]_T(k_B + [A])}$$

$$\frac{(k_C + [A])}{(k_B + [A])}$$

(Enrichment Factor)

The above factor is termed the "Enrichment Factor". The ratio of the total components is multiplied by this factor to calculate the ratio of the bound components, or the enrichment of B over C. The maximum enrichment factor is $k_C/k_B$, when the [A] is significantly smaller than $k_c$ or $k_B$.

The enrichment is limited by the ratio of binding constants. To enrich a scarce protein that is bound 100 times as strongly as its competitors, the ratio of that protein to its competitors is increased by 1 million with 3 enrichments. To enrich a protein that only binds twice as strongly than its competitors, 10 enrichment cycles would gain only an enrichment of ~1000.

The following Example illustrate various embodiments of the present invention and are not intended in any way to limit the invention.

## EXAMPLE 1: PRODUCTION OF THE SATA

One skilled in the art will understand that the SATA can be produced in a number of different ways. For example, in a preferred embodiment, three fragments (Fig 1) are purchased from a commercial source (i.e. Dharmacon Research Inc., Boulder, CO). Modified bases and a fragment 3 with a pre-attached puromycin on its 3' end and a $PO_4$ on its 3' end are included, all of which are available commercially. Three fragments are used to facilitate manipulation of the fragment 2 in forming the monoadduct.

Yeast tRNAA1a or yeast tRNAPhe is used; however, sequences can be chosen widely from known tRNA's. Preferably, sequences with only a limited number of U's in the portion that corresponds to the fragment 2 are used. Using a sequence with only a few U's is not necessary because psoralen preferentially binds 5'UA3' sequences (Thompson J. F, et al Biochemistry 21:1363). However, there would be less doubly adducted product to purify out if such a sequence was used.

The fragment 2 is used in a helical conformation to induce the psoralen to intercalate. Accordingly, a complementary strand is required. RNA or DNA is used, and a sequence, such as poly C to one or both ends, is added to facilitate separation and removal after monoadduct formation is accomplished.

The fragment 2 and the cRNA are combined in buffered 50 mM NaCl solution. The Tm is measured by hyperchromicity changes. The two molecules are re-annealed and incubated for 1 hour with the selected psoralen at a temperature ~10°C less than the Tm. The psoralen is selected based upon the sequence used. For instance, a relatively insoluble psoralen, such as 8 MOP, has a higher sequence stringency but may need to be replenished. A more soluble psoralen, such as AMT, has less stringency but will fill most sites. Preferably, HMT is used. If a fragment 2 is chosen that contains more non-target U's, a greater stringency is desired. Decreasing the temperature or increasing ionic strength by adding $Mg^{++}$ is also used to increase the stringency.

Following incubation, psoralen is irradiated at a wavelength greater than approximately 400 nm. The irradiation depends on the wavelength chosen and the psoralen used. For instance, approximately 419 nm 20-150 J/cm2 is preferably used for HMT. This process will result in an almost entirely furan sided monoadduct.

### Purification of Monoadduct

The monoadduct is then purified by HPLC as described in Sastry et al, J. Photochem. Photobiol. B Biol. 14: 65-79. The fact that fragment 2 is separate from fragment 3 facilitates the purification step because, generally, purification of monoadducts ≥25 mer is difficult (Spielmann et al. PNAS 89: 4514-4518).

### Ligation of Fragment 2 and 3

The fragment 2 is ligated to the fragment 3 using T4 RNA ligase. The puromycin on the 3' end acts as a protecting group this is done as per Romaniuk and Uhlenbeck, Methods in Enzymology 100:52-59 (1983). Joining of fragment 2+3 to the 3' end of fragment 1 is done according to the methods described in Uhlenbeck, Biochemistry 24: 2705-2712 (1985). Fragment 2+3 is 5' phosphorylated by polynucleotide kinase and the two half molecules are annealed.

In an alternative method, significant quantities of furan sided monoadducted U will be formed by hybridizing poly UA to itself and irradiating as above. The poly UA will then be enzymatically digested to yield furan sided U which will be protected and incorporated into a tRNA analog by nucleoside phosphoramidite methods. Other methods of forming the psoralen monoadducts include the methods described in Gamper et al., J. Mol. Biol. 197: 349 (1987); Gamper et al., Photochem. Photobiol. 40:29, 1984; Sastry et al, J. Photochem. Photobiol. B Biol. 14:65-79; Spielmann et al. PNAS 89:4514-4518; U.S. Patent Number 4,599,303.

SATAs generated by the methods described above will read UAG (anticodon CUA). Additionally, UAA or UGA will be also be used. In various embodiments, any message that has the stop codon that is selected as the "linking codon" is used.

## EXAMPLE 2: PRODUCTION OF PSORALENATED FURAN SIDED MONOADDUCTS FROM CUAGAΨCUGGAGG RNA FRAGMENTS

*UV Light Exposure Of RNA: DNA Hybrids*

Equal volumes of 3 ng/ml RNA:cRNA hybrid segments and of 10 μg/ml HMT both comprised of 50mM NaCl are transferred into a new 1.5 ml capped polypropylene microcentrifuge tube and incubated at 37°C for 30 minutes in the dark. This was then transferred onto a new clean culture dish. This is positioned in a photochemical reactor (419 nm peak Southern New England Ultaviolet Co.) at a distance of about 12.5 cm so that irradiance is -6.5 mW/cm2 and irradiated for 60-120 minutes.

*Removal of Low Molecular Weight Protoproducts*

100μl of chloroform-isoamyl alcohol (24:1) is pipetted and mixed by vortex.. Mixture is centrifuged for 5 minutes at 15000 xg in a microcentrifuge tube. The chloroform-isoamyl alcohol layer is removed with a micropipet. The chloroform-isoamyl alcohol extraction is repeated once again. Clean RNA is precipitated out of the solution.

*Alcohol Precipitation*

Two volumes (~1000 μl) ice cold absolute ethanol is added to the mixture. The tube is centrifuged for 15 minutes at 15,000xg in a microcentrifuge. The supernatant is decanted and discarded and the precipitated RNA is redissolved in 100μl DEPC treated water then re-exposed to the RNA+8-MOP.

*Isolation of the Psoralentated RNA Fragments Using HPLC*

All components, glassware and reagents are prepared so that they are RNAase free. The HPLC is set up with a Dionex DNA PA-100 package column. The psoralenated RNA:DNA hybrid is warmed to 4°C. The psoralenated RNA is applied to HPLC followed by olignucleotide analysis, as described in the following section entitled "Olignucleotide Analysis by HPLC." The collected fractions will represent:

a) 5'CUAGAΨCUGGAGG3', where Ψ is pseudouridine (SEQ ID NO: 1)

b) Furan sided 5'CUPsoralenAGAΨCUGGAGG3' monoadducts (SEQ ID NO: 2)

c) 5'XXXXXCCUCCAGAUCUAGXXXXX3' (SEQ ID NO: 3)

d) 5'XXXXXCCUCCAGAUCUPsoralenAGXXXXX3' (SEQ ID NO: 4)

The fractions are stored at 4°C in new, RNAase free snapped microcentrifuge tubes and stored at -20°C if more than four weeks of storage is required.

*Identification of the RNA Fragments Represented by Each Peak Fraction Collected by HPLC Using Polyacrylamide Gel Electrophoresis (PAGE)*

The electrophoresis unit is set up in a 4°C refrigerator. A gel is selected with a 2 mm spacer. Each 5 μl of HPLC fraction is diluted to 10 μl with Loading Buffer. 10 μl of each diluted fraction is loaded into appropriately labeled sample wells. The tracking dye is loaded in a separate lane and electrophoresis is run as described in the following section entitled "Polyacrylamide Gel Electrophoresis (PAGE) of Psoralenated RNA Fragments." After the electrophoresis run is complete, the electrophoresis is stopped when the tracking dye has reached the edge of the gel. The apparatus is disassembled. The gel-glass panel unit is placed on the UV light box. UV lights are turned on. The RNA bands are identified. The bands will appear as denser shadows under UV lighting conditions.

*Extraction of the RNA From the Gel*

Each band is excised with a new sterile and RNAase free scalpel blade and transferred into a new 1.5 ml snap capped microcentrifuge tube. Each gel is crushed against the walls of the microcentrifuge tubes with the side of the scalpel blade. A new blade is used for each sample. 1.0 ml of 0.3M sodium acetate is added to each tube and eluted for at least 24 hours at 4°C. The eluate is transferred to a new 0.5 ml snap capped polypropylene microcentrifuge tube with a micropipet. A new RNAase free pipette tip is used for each tube and the RNA with ethanol is precipitated out.

*Ethanol Precipitation*

Two volumes of ice cold ethanol is added to each eluate then centrifuged at 15,000 xg for 15 minutes in a microcentrifuge. The supernatants are discharged and the precipitated RNA is re-dissolved in 100 μl of DEPC treated

DI water. The RNA is stored in the microcentrifuge tubes at 4°C until needed. The tubes are stored at -20°C if storage is for more than two weeks. The following is the assumed order of rate of migration for each fragment in order from fastest to slowest:

a) 5'CUAGAΨCUGGAGG3

b) Furan sided 5'CUPsoralenAGAΨCUGGAGG3 monoadducts.

c) 5'XXXXXCCUCCAGAUCUAGXXXXX3'

d) 5'XXXXXCCUCCAGAUCUPsoralenAGXXXXX3'

The tubes are labeled containing the remainder of each fraction with the presumed chemical sequence and stored at -20°C.

**Ethanol Precipitation of RNA**

RNA oligonucleotide fragments are precipitated, and all glassware is cleaned to remove any traces of RNase as described in the following section entitled "Inactivation of RNases on Equipment, Supplies, and in Solutions." All solutions are stored in RNAase free glassware and introduction of nucleases is prevented. Absolute ethanol is stored at 0°C until used. Micropipetors are used to add two volumes of ice cold ethanol to nucleic acids that are to be precipitated in microcentrifuge tubes. Capped microcentrifuge tubes are placed into the microfuge and spun at 15,000 xg for 15 minutes. The supernatant is discarded and precipitated RNA is re-dissolved in DEPC treated DI-water. RNA is stored at 4°C in microcentrifuge tubes until ready to use.

**Ligation of RNA Fragments 2 and 3**

All glassware is cleaned to remove any traces of RNase as described in the following section entitled "Inactivation of RNases on Equipment, Supplies, and in Solutions." The following is added to a new 1.5 ml polypropylene snap capped microcentrifuge tube using a 100-1000 μl pipet and a new sterile pipet tip is used for each solution:

| | |
|---|---|
| Fragment 2 (3.0nM) | 125.0 μl |
| Fragment 3 (3.0nM) | 125.0 μl |
| Reaction buffer | 250.0μl |
| RNA T4 ligase (9-12U/ml) | 42 μl |

| Reaction Buffer | |
|---|---|
| RNase free DI-water | 90.00ml |
| Tris-HCl (50mM) | 0.79g |
| MgCl2 (10mM) | 0.20g |
| DTT (5mM) | 0.078g |
| ATP (1mM) | 0.55g |
| pH to 7.8 with HCL | |
| RNase free DI-water | QS to 100.00ml |

The mixture is gently mixed and the RNA is melted by incubating the mixture at 16°C for one hour in a temperature controlled refrigerated chamber. RNA is precipitated out of the solution immediately after the incubation is completed.

*Alcohol Precipitation*

Two volumes (~1000 μl) of ice cold absolute ethanol are added to the reaction mixture. The microcentrifuge tube is placed in a microcentrifuge at 15,000 xg for 15 minutes. The supernate is decanted and discarded and the precipitated RNA is re-dissolve in 100 μl DEPC treated water. The mixture is electrophoresed as described in the following section entitled "Polyacrylamide Gel Electrophoresis (PAGE) of Psoralenated RNA Fragments." The following is the assumed order of rate of migration for each fragment in order from fastest to slowest:

a) Frag. 2
5'CUAGAΨCUGGAGG3'-OH
Psoralen

b) Frag. 3
5'UCCUGUGTΨCGAUCCACAGAAUUCGCACC-Puromycin (SEQ ID NO: 5)

c) Frag 2+3
5'CUAGAYCUGGAGGUCCUGUGTΨCGAUCCACAGAAUUCGCACCPuromy cin (SEQ ID NO: 6)
Psoralen

Each fraction is isolated by UV shadowing, the bands are cut out, the RNAs are eluted from the gels and the RNA elute is precipitated out as described in the following section entitled "Polyacrylamide Gel Electrophoresis (PAGE) of Psoralenated RNA Fragments."
The ligation procedure is repeated with any residual unligated fragment 2 and 3 fractions.
The ligated fractions 2 and 3 are pooled and stored in a small volume of RNase free DI-water at 4°C.

## Ligation of RNA Fragment 1 with Fragment 2+3

All glassware is cleaned to remove any traces of RNase as described in the following section entitled "Inactivation of RNases on Equipment, Supplies, and in Solutions." The following is added to a new 1.5 ml polypropylene snap capped microcentrifuge tube. A 100-1000 $\mu$l pipet and new tip is used for each solution:

| | |
|---|---|
| Fragment 2+3 (3.0nM) | 125.0$\mu$l |
| Reaction buffer | 250.0$\mu$l |
| T4 Polynucleotide Kinase(5-10U/ml) | 1.7$\mu$l |

| Reaction Buffer | |
|---|---|
| RNase free DI-water | 90.00ml |
| Tris-HCl (40mM) | 0.63g |
| MgC12 (10mM) | 0.20g |
| DTT (5mM) | 0.08g |
| ATP (1mM) | 0.006g |
| pH to 7.8 with HCL | |
| RNase free DI-water | QS to 100.00ml |

The RNA is gently mixed then melted by heating the mixture to 70°C for 5 minutes in a heating block. The mixture is cooled to room temperature over a two hour period and the RNAs is allowed to anneal in a tRNA configuration. The RNA is precipitated out of the solution.

*Alcohol Precipitatio*n

Two volumes (~1000 $\mu$l) if ice cold absolute ethanol are added to the reaction mixture. The microcentrifuge tube is placed in a microcentrifuge at 15,000 xg for 15 minutes. The supernate is decanted and discarded and the precipitated RNA is re-dissolved in 100 $\mu$l DEPC treated water. The mixture is electrophoresed as described in the following section entitled "Polyacrylamide Gel Electrophoresis (PAGE) of Psoralenated RNA Fragments." The following is the assumed order of rate of migration for each fragment in order from fastest to slowest:

a) Frag. 1
5'GCGGAUUUAGCUCAGDDGGGAGAGCGCCAGACU3'

b) Frag 2+3
5'CUAGAYCUGGAGGUCCUGUGTΨCGAUCCACAGAAUUCGCACCPuromy cin
Psoralen

c) Frag. 1+2+3

5'GCGGAUUUAGCUCAGDDGGGAGAGCGCCAGACUCUAGAΨCUGGAGG    UC...CUGUGTΨCGAUCCACAGAAUUCGCACCPuromycin (SEQ ID NO: 7)

Psoralen

Each fraction is isolated by UV shadowing, the bands are cut out, the RNAs are eluted from the gels and the RNA elute is precipitated out as described in the following section entitled "Polyacrylamide Gel Electrophoresis (PAGE) of Psoralenated RNA Fragments." The ligation procedure is repeated with the unligated Fragment 1 and the 2+3 Fraction. The ligated fractions 2 +3 are pooled and stored in a small volume of RNase free DI-water at 4°C.

*Final RNA Ligation*

The following is added to a new 1.5ml polypropylene snap capped microcentrifuge tube. A 100-1000 $\mu$l pipet and new tip is used for each solution:

| | |
|---|---|
| Fragment 1+2+3 (3.0nM) | 250 $\mu$l |
| reaction buffer | 250 $\mu$l |
| RNA T4 ligase (44 $\mu$g/ml) | 22$\mu$g |

The mixture is incubated at 17°C in a temperature controlled refrigerator for 4.7 hours. Immediately after the incubation the tRNA is precipitated out as described in step 6.2 above and the tRNA is isolated by electrophoresis as described in the following section entitled ''Polyacrylamide Gel Electrophoresis (PAGE) of Psoralenated RNA Fragments." The tRNA is pooled in a small volume of RNase free water and stored at 4°C for up to two weeks or stored at -20°C for periods longer than two weeks.

**Polyacrylamide Gel Electrophoresis (PAGE) of Psoralenated RNA Fragments**

*Acrylamide Gel Preparation*

All reagents and glassware must be RNAase free as described in the following section entitled "Inactivation of RNases on Equipment, Supplies, and in Solutions." The gel apparatus is assembled to produce a 4 mm thick by 20 cm x 42 cm square gel. 29 parts acrylamide with 1 part ammonium crosslinker are mixed at room temperature with the appropriate amount of acrylamide solution in an RNAase free, thick walled Erlenmeyer flask.

| Acrylamide Solution | |
|---|---|
| urea (7M) | 420.42 g |
| TBE (1X) | QS to 1L |

| 5X TBE | |
|---|---|
| 0.455 M Tris-HCl | 53.9g |
| 10mM EDTA | 20ml of 0.5 M |
| RNAase free DI water | 900ml |
| pH with boric acid to | pH 9 |
| QS with RNAase free DI water to | 1L |

The mixture is degassed with vacuum pressure for one minute. The appropriate amount of TEMED is added, mixed gently, and then the gel mixture is poured between the glass plates to within 0.5 cm of the top. The comb is immediately inserted between the glass sheets and into the gel mixture. An RNAase free gel comb is used. The comb should produce wells for a 5 mm wide dye lane and 135 mm sample lanes. The gel is allowed to polymerize for about 30-40 minutes then the comb is carefully removed. The sample wells are rinsed out with a running buffer using a micropipet with a new pipet tip. The wells are then filled with running buffer.

*Sample Preparation*

An aliquot of the sample is suspended in loading buffer in a snap capped microcentrifuge tube and vortex mixed. Indicator dye is not added to the sample.

| Loading Buffer | |
| --- | --- |
| Urea (7M) | 420.42 g |
| Tris HCl (50mM) | 7.85 g |
| QS with RNAase free D-H2O | to 1L |

*Electrophoresis run*

The maximum volume of RNA/loading buffer solution is loaded into the 135 mm sample wells and the appropriate volume of tracking dye in 5 mm tracking lane. The samples are electrophoresed in a 5°C refrigerator. The electrophoresis is stopped when the tracking dye has reached the edge of the gel. Disassemble the apparatus. Glass panels are not removed the from the gel. The gel-glass panel unit is placed on a UV light box. With UV filtering goggles in place, the UV lights are turned on. The RNA bands are identified. They appear as denser shadows under UV lighting conditions. The RNA is extracted from the gel. Each bands is excised with a new sterile and RNAase free scalpel blade and each band is transferred into a new 1.5 ml snap capped microcentrifuge tube. Each gel is crushed against the walls of the microcentrifuge tubes with the side of the scalpel blade. A new blade is used for each sample. 1.0 ml of 0.3M sodium acetate is added to each tube and eluted for at least 24 hours at 4°C. The eluate is transferred to a new 0.5 ml snap capped polypropylene microcentrifuge tubes with a micropipet with a new RNAase free pipet tip for each tube. Two volumes of ice cold ethanol is added to each eluate, then centrifuged at 15,000xG for 15 minutes in a microcentrifuge. The supernatants are discarded and the precipitated RNA is redissolved in 100 $\mu$l of DEPC treated DI water. The RNA is stored in the microcentrifuge tubes at 4°C until needed.

**Olignucleotide Analysis by HPLC**

HPLC purification of the RNA oligonucleotides is best effected using anion exchange chromatography. Either the 2'-protected or 2'-deprotected forms can be chromatographed. The 2'-protected form offers the advantage of minimizing secondary structure effects and provides resistance to nucleases. If the RNA is fully deprotected, sterile conditions are required during purification.

*Deprotection of 2'-Orthoester protected RNA*

The tubes are centrifuged at 15,000xg for 30 seconds or until the RNA pellet is at the bottom. 400 $\mu$l of pH 3.8 deprotection buffer is added to each tube of RNA.

*Deprotection Buffer*

Acetic acid (100mM) is adjusted to pH 3.8 with tetramethylethylenediamine (TEMED). The pellet is completely dissolved in the buffer by drawing in and out of a pipette. The tubes are vortexed for 10 seconds and centrifuged at 15,000xg. The tubes are incubated in a 60 °C water bath for 30 minutes. The samples are lyophilized before use.

*HPLC Column Conditions*

A 4 x 250 mm column (DNAPAC PA, No. 043010) packed with Dionex (800)-DIONEX-0 (346-6390), with a capacity of 40 optical density units (ODU) at 260 nm is installed. The column temperature is set to 54°C. The injection volume is adjusted such that 5 $\mu$l produces approximately 0.20 ODU

*Elution buffers*

| Condition | Buffer A | Buffer B |
|---|---|---|
| Sodium perchlorate | (5mM) 2.8 g | (300mM)168.0 g |
| Tris-HCl | 2.4 g | 2.4 g |
| Acetonitrile (2%) | 80.0 ml | 80.0 ml |
| DI Water | 3900 ml | 900 ml |
| Adjusted pH | 8.0 with HCL | 8.0 with HCL |
| q.s. | 4000 ml | 4000 ml |

*HPLC Gradient*

A 30% to 60% gradient of Buffer B for oligos 17-32 base pairs long is provided:

| Time (minutes) | Flow (ml/min) | %A | %B | Curve |
|---|---|---|---|---|
| 0 | 1.5 | 100 | 0 | * |
| 1 | 1.5 | 100 | 0 | 6 |
| 3 | 1.5 | 70* | 30* | 6 |
| 15 | 1.5 | 40* | 60* | 6 |
| 15.5 | 2.5 | 0 | 100 | 6 |
| 17 | 2.5 | 0 | 100 | 6 |
| 17.25 | 2.5 | 100 | 0 | 6 |
| 23 | 2.5 | 100 | 0 | 6 |
| 23.1 | 1.5 | 100 | 0 | 6 |
| 24 | 1.5 | 100 | 0 | 6 |
| 25 | 0.1 | 100 | 0 | 6 |
| * % values that can be changed to modify the gradient. Typical gradients are 0-30%, 20-50%, 30-60%, and 40-70% of Buffer B. | | | | |

*Gradient selection*

The gradient is selected based upon the number of bases, as follows:

| Number of bases | Gradient |
|---|---|
| 0-5 | 0-30 |
| 6-10 | 10-40 |
| 11-16 | 20-50 |
| 17-32 | 30-60 |
| 33-50 | 40-70 |
| >50 | 50-80 |

After HPLC, the target samples are collected and the KNA concentration is determined with a spectrophotometer at 260 nm. The samples are stored at -70 °C.

**Inactivation of RNases on Equipment, Supplies, and in Solutions**

Glassware is treated by baking at 180°C for at least 8 hours. Plasticware is treated by rinsing with chloroform. Alternatively, all items are soaked in 0.1% DEPC.

*Treatment with 0.1 % DEPC*

0.1% DEPC is prepared. DI water is filtered through a $0.2\mu M$ membrane filter. The water is autoclaved at 15 psi for 15 minutes on a liquid cycle. 1.0g (wt/v) DEPC/liter of sterile filtered water is added.

*Glass and Plasticware*

All glass and plasticware is submerged in 0.1% DEPC for two hours at 37°C. The glassware is rinsed at least 5X with sterile DI water. The glassware is heated to 100°C for 15 minutes or autoclaved for 15 minutes at 15 psi on a liquid cycle.

*Electrophoresis Tanks Used for Electrophoresis of RNA*

Tanks are washed with detergent, rinsed with water then ethanol and air dried. The tank is filled with 3% (v/v) hydrogen peroxide (30ml/L) and left standing for 10 minutes at room temperature. The tank is rinsed at least 5 times with DEPC treated water.

*Solutions*

All solutions are made using Rnase free glassware, plastic ware, autoclaved water, chemicals reserved for work with RNA and RNase free spatulas. Disposable gloves are used. When possible, the solutions are treated with 0.1 % DEPC for at least 12 hours at 37°C and then heated to 100°C for 15 minutes or autoclaved for 15 minutes at 15 psi on a liquid cycle.

**RNA Translation**

2 $\mu$l of gastroinhibitory peptide (GIP) mRNA at a concentration of 20 $\mu$l/ml is placed in a 250 $\mu$l snapcap polypropylene microcentrifuge tube. 35 $\mu$l of rabbit reticulocyte lysate (available commercially from Promega) is added. 1 $\mu$l of amino acid mixture which does not contain methionine (available commercially from Promega) is added. 1 $\mu$l of 35S methione or unlabeled methionine is added. Optionally, 2 ml of luciferase may be added to some tubes to serve as a control.

SATA is added to the experimental tubes. Control tubes which do not contain SATA are also prepared. The quantity of SATA used is approximately between 0.1 $\mu$g to 500 $\mu$g, preferably between 0.5 $\mu$g to 50 $\mu$g. 1 $\mu$l of Rnasin at 40 units/ml is added. Nuclease free water is added to make a total volume of 50 $\mu$l.

For proteins greater than approximately 150 amino acids, the amount of tRNA may need to be supplemented. For example, approximately 10 - 200 $\mu$g of tRNA may be added. In general, the quantity of the SATA should be high enough to effectively suppress stop or pseudo stop codons. The quantity of the native tRNA must be high enough to out compete the SATA which does not undergo dynamic proofreading under the action of elongation factors.

Each tube is immediately capped, parafilmed and incubated for the translation reactions at 30°C for 90 minutes. The contents of each reaction tube is transferred into a 50 $\mu$l quartz capillary tube by capillary action. The SATA is crosslinked with mRNA by illuminating the contents of each tube with 2-10 J/cm2 ~350 nm wavelength light, as per Gasparro et al. (Photochem. Photobiol. 57:1007 (1993)). Following photocrosslinking, the contents of each tube is transferred into a new snapcap microfuge tube. The ribosomes are dissociated by chelating the calcium cations by adding 2 $\mu$l of 10 mM EDTA to each tube. Between each step. each tube is gently mixed by stirring each component with a pipette tip upon addition.

The optimal RNA for a translation is determined prior to performing definitive experiments. Serial dilutions may be required to find the optimal concentration of mRNA between 5-20 $\mu$g/ml.

| Reagent | 1 | 2 | 3 | 4 |
|---|---|---|---|---|
| Rabbit reticulocyte lysate (35$\mu$l) | + | + | + | + |
| Amino acid mixture minus methionine (1$\mu$l of 1 mM) | + | + | + | + |

(continued)

| Reagent | 1 | 2 | 3 | 4 |
|---|---|---|---|---|
| $^{35}$S Methionine (1μl of 1,200Ci/mmol) | + | - | - | + |
| Methionine (unlabeled) | - | + | + | - |
| GIP mRNA (2μl of 20 μg/ml) | + | - | - | - |
| $^{32}$P GIP mRNA (2 μl of 20 μg/ml) | - | + | + | - |
| Rnasin (1 μl of 40 U/μl) | + | + | + | + |
| SATA | - | - | - | |
| Water, nuclease free (q.s. to 50 μl) | + | + | + | + |

SDS-Page electrophoresis is performed on each sample, as described above. Autoradiography on the gel is performed, as described by Sambrook et. al., Molecular Cloning, A Laboratory Manual, 2nd ed., Coldspring Harbor Press (1989).

While a number of preferred embodiments of the current invention and variations thereof have been described in detail, other modifications and methods of use will be readily apparent to those of skill in the art.

## Claims

1. A method for selecting a desired protein or nucleic acid molecule comprising:

   providing an in vitro translation system
   providing at least two candidate mRNA molecules, wherein at least one of said mRNA molecules is untranslatable beyond a particular codon selected from the group consisting of a stop codon, a codon for which tRNAs having an anticodon complementary thereto are absent or substantially depleted in said in vitro translation system, and a terminal codon of a reading frame of said mRNA molecule;
   translating at least two of said candidate mRNA molecules in said in vitro translation system to generate at least one translated protein;
   linking at least one of said candidate mRNA molecules to its corresponding translated protein via a crosslink at said particular codon to form at least one cognate pair;
   selecting one or more of said cognate pairs based upon the properties of said translated protein or said mRNA molecule; and
   selecting said translated protein or said desired nucleic acid molecule comprising identifying a molecule selected from the group consisting of an mRNA molecule of said selected cognate pair, a nucleic acid molecule complementary to said mRNA molecule and a nucleic acid molecule homologous to said mRNA molecule.

2. The method of Claim 1, wherein at least one of said candidate mRNA molecules and at least one said translated protein is linked by a tRNA molecule selected from the group consisting of tRNA, modified tRNA and tRNA analogues.

3. The method of Claim 2, wherein said tRNA molecule is connected to said translated protein by ribosomal peptidyl transferase.

4. The method of Claim 2, wherein said tRNA molecule is connected to the mRNA through an ultraviolet-induced cross link between the anticodon of said tRNA molecule and the codon of the RNA message.

5. The method of Claim 2, wherein said tRNA molecule is connected to the mRNA by thiouracil.

6. The method of Claim 2, wherein said tRNA molecule is connected to the mRNA by a psoralen cross link.

7. The method of Claim 2, wherein said tRNA molecule is connected to the mRNA by using a psoralen monoadduct.

8. The method of Claim 7, wherein said psoralen monoadduct is placed on said mRNA.

9. The method of Claim 7, wherein said psoralen monoadduct is placed on an anticodon of said tRNA molecule.

10. The method of Claim 7, wherein said psoralen monoadduct is placed on a stop anticodon of said tRNA molecule.

11. The method of Claim 7, wherein said psoralen monoadduct is generated by forming crosslinks and photo reversing said crosslinks or by using visible light.

12. The method of Claim 2, wherein said tRNA molecule has a stable peptide acceptor.

13. The method of Claim 2, wherein said tRNA molecule is modified to accept a peptide chain and hold said chain in a stable manner, such that peptidyl transferase cannot detach it.

14. The method of Claim 2, wherein said tRNA molecule is modified by using a bond which binds to the ribosome, accepts the peptide chain, and then does not act as a donor in the next transpeptidation.

15. The method of Claim 14, wherein said bond is selected from the group consisting of a 2'ester on a 3'deoxy adenosine and an amino acyl tRNA$_{ox-red}$.

16. The method of Claim 2, wherein an amino acid or amino acid analog is attached to the 3'end of said tRNA molecule by a stable bond to generate a stable amino acyl tRNA analogue.

17. The method of Claim 1, wherein said translating is performed in vitro.

18. The method of Claim 1, wherein said translating is performed in situ.

19. The method of Claim 1, wherein said cognate pair is selected based upon ligand binding.

20. The method of Claim 1, wherein selecting said desired nucleic acid molecule comprises:

   having an array of nucleic acids, wherein said nucleic acids are placed in a predetermined position;
   hybridizing at least one of said cognate pairs onto said array; and
   identifying the mRNA based upon its reaction to a protein identifier.

21. The method of Claim 20, wherein said reaction to a protein identifier is selected from the group consisting of ligand binding, immuno-precipitation and enzymatic reactions.

22. A method for evolving a desired protein sequence comprising:

   selecting at least one first cognate pairs based upon at least one desired characteristic of a translated first protein or said mRNA molecule according to the method of Claim 1;
   recovering at least one of said first cognate pairs with said desired characteristic to generate at least one recovered cognate pair;
   amplifying a first nucleic acid component of one or more of said recovered cognate pairs;
   producing at least one second nucleic acid component comprising at least one of said first nucleic acid components with one or more variations;
   producing at least one second protein by translating at least one of said second nucleic acid components;
   linking at least one of said second proteins with at least one of said second nucleic acid components to generate one or more second cognate pairs; and
   obtaining the desired protein sequence by re-selecting one or more of said second cognate pairs based upon at least one desired property, wherein said desired property is the same or different than said desired characteristic.

23. The method of Claim 22, wherein said desired characteristic is selected from the group consisting of binding properties, enzymatic reactions and chemical modifications.

**Patentansprüche**

1. Verfahren zum Auswählen eines erwünschten Protein- oder Nukleinsäuremoleküls, umfassend:

   Bereitstellen eines in vitro Translationssystems;
   Bereitstellen von zumindest zwei Kandidaten mRNA Molekülen, wobei zumindest eines der mRNA Moleküle über ein bestimmtes Codon hinaus nicht translatiert werden kann, das ausgewählt ist aus der Gruppe bestehend aus einem Stopcodon, einem Codon für welches tRNAs mit einem dazu komplementären Anticodon in dem in vitro Translationssystem abwesend oder im Wesentlichen verringert sind, und ein Endcodon eines Leserasters des mRNA Moleküls;

Translatieren von zumindest zwei der Kandidaten mRNA Moleküle in dem in vitro Translationssystem, um zumindest ein translatiertes Protein zu erzeugen;

Vernetzen von zumindest einem der Kandidaten mRNA Moleküle mit seinem entsprechenden translatierten Protein über ein Vernetzen an dem bestimmten Codon, um zumindest ein verwandtes Paar zu bilden.

Auswählen von einem oder mehreren der verwandten Paare basierend auf den Eigenschaften des translatierten Proteins oder des mRNA Moleküls; und

Auswählen des translatierten Proteins oder des erwünschten Nukleinsäuremoleküls, umfassend Identifizieren eines Moleküls ausgewählt aus der Gruppe bestehend aus einem mRNA Molekül von dem ausgewählten, verwandten Paar, einem zu dem mRNA Molekül komplementären Nukleinsäuremolekül und einem zu dem mRNA Molekülen homologen Nukleinsäuremolekül.

2. Verfahren nach Anspruch 1, wobei zumindest eines der Kandidaten mRNA Moleküle und zumindest eines der translatierten Proteine durch ein tRNA Molekül vernetzt ist, welches ausgewählt ist aus der Gruppe bestehend aus tRNA, modifizierter tRNA und tRNA Analogen.

3. Verfahren nach Anspruch 2, wobei das tRNA Molekül mit dem translatierten Protein durch ribosomale Peptidyltransferase verbunden ist.

4. Verfahren nach Anspruch 2, wobei das tRNA Molekül mit der mRNA durch ein ultraviolett-induziertes Vernetzen zwischen dem Anticodon des tRNA Moleküls und dem Codon der RNA Botschaft verbunden ist.

5. Verfahren nach Anspruch 2, wobei das tRNA Molekül mit der mRNA durch Thiouracil verbunden ist.

6. Verfahren nach Anspruch 2, wobei das tRNA Molekül mit der mRNA durch ein Psoralen Vernetzen verbunden ist.

7. Verfahren nach Anspruch 2, wobei das tRNA Molekül mit der mRNA durch Verwenden eines Psoralen Monoaddukts verbunden ist.

8. Verfahren nach Anspruch 7, wobei das Psoralen Monoaddukt auf der mRNA platziert ist.

9. Verfahren nach Anspruch 7, wobei das Psoralen Monoaddukt auf einem Anticodon des tRNA Moleküls platziert ist.

10. Verfahren nach Anspruch 7, wobei das Psoralen Monoaddukt auf einem Stop-Anticodon des tRNA Moleküls platziert ist.

11. Verfahren nach Anspruch 7, wobei das Psoralen Monoaddukt durch Bilden von Vernetzungen und Fotoreversieren der Vernetzungen oder durch Verwenden von sichtbarem Licht erzeugt wird.

12. Verfahren nach Anspruch 2, wobei das tRNA Molekül einen stabilen Peptidakzeptor aufweist.

13. Verfahren nach Anspruch 2, wobei das tRNA Molekül für ein Annehmen einer Peptidkette und Halten der Kette auf eine stabile Art derart modifiziert ist, dass Peptidyltransferase es nicht lösen kann.

14. Verfahren nach Anspruch 2, wobei das tRNA Molekül durch Verwenden einer Bindung modifiziert ist, welche an das Ribosom bindet, die Peptidkette annimmt, und anschließend nicht als ein Donor in der nächsten Transpeptidierung wirkt.

15. Verfahren nach Anspruch 14, wobei die Bindung ausgewählt ist aus der Gruppe bestehend aus einem 2' Ester auf einem 3'Desoxyadenosin und einem Aminoacyl tRNA$_{ox-red}$

16. Verfahren nach Anspruch 2, wobei eine Aminosäure oder ein Aminosäureanaloges an dem 3' Ende des tRNA Moleküls durch eine stabile Bindung angebracht ist, um ein stabiles Aminoacyl tRNA Analog zu erzeugen.

17. Verfahren nach Anspruch 1, wobei das Translatieren in vitro durchgeführt wird.

**18.** Verfahren nach Anspruch 1, wobei das Translatieren in situ durchgeführt wird.

**19.** Verfahren nach Anspruch 1, wobei das verwandte Paar basierend auf Ligandenbindung ausgewählt wird.

**20.** Verfahren nach Anspruch 1, wobei Auswählen des erwünschten Nukleinsäuremoleküls umfasst:

Aufweisen einer Anordnung von Nukleinsäuren, wobei die Nukleinsäuren in einer bestimmten Position platziert sind;
Hybridisieren von zumindest einem der verwandten Paare auf der Anordnung; und
Identifizieren der mRNA basierend auf ihrer Reaktion mit einem Proteinidentifikator.

**21.** Verfahren nach Anspruch 20, wobei die Reaktion mit einem Proteinidentifikator ausgewählt ist aus der Gruppe bestehend aus Ligandenbindung, Immuno-Präzipitation und enzymatischen Reaktionen.

**22.** Verfahren zum Entwickeln einer erwünschten Proteinsequenz, umfassen:

Auswählen von zumindest einem ersten verwandten Paar basierend auf zumindest einem erwünschten Merkmal eines translatierten ersten Proteins oder des mRNA Moleküls gemäß dem Verfahren nach Anspruch 1;
Wiedererlangen von zumindest einem der ersten verwandten Paare mit dem erwünschten Merkmal, um zumindest ein wiedererlangtes verwandtes Paar zu erzeugen;
Amplifizieren einer ersten Nukleinsäurekomponente von einem oder mehreren der wiedererlangten verwandten Paare;
Erzeugen von zumindest einer zweiten Nukleinsäurekomponente, welche zumindest eine der ersten Nukleinsäurekomponenten mit einer oder mehreren Variationen umfasst;
Erzeugen von zumindest einem zweiten Protein durch Translatieren von zumindest einer der zweiten Nukleinsäurekomponenten;
Vernetzen von zumindest einem der zweiten Proteine mit zumindest einer der zweiten Nukleinsäurekomponenten, um eines oder mehrere zweite verwandte Paare zu erzeugen; und
Erhalten der erwünschten Proteinsequenz durch erneutes Auswählen von einer oder mehreren der zweiten verwandten Paare basierend auf zumindest einer erwünschten Eigenschaft, wobei die erwünschte Eigenschaft die gleiche oder verschieden von dem erwünschten Merkmal ist.

**23.** Verfahren nach Anspruch 22, wobei das erwünschte Merkmal ausgewählt ist aus der Gruppe bestehend aus Bindeeigenschaften, enzymatischen Reaktionen und chemischen Modifikationen.

**Revendications**

**1.** Une méthode pour sélectionner une molécule de protéine ou d'acide nucléique désirée comprenant :

fournir un système de translation in vitro ;
fournir au moins deux molécules mRNA candidates, dans lesquelles au moins une desdites molécules mRNA est non translatable au-delà d'un codon particulier sélectionné à partir du groupe consistant en un codon d'arrêt, un codon pour lequel les tRNA ayant un codon complémentaire à celui-ci sont absents, ou un codon essentiellement épuisé dans ledit système in vitro et un codon terminal d'un cadre de lecture de ladite molécule mRNA ;
translater au moins deux desdites molécules mRNA candidates dans ledit système de translation in vitro pour générer au moins une protéine translatée ;
lier au moins une desdites molécules mRNA candidates à sa protéine translatée correspondante par une réticulation audit codon particulier pour former au moins une paire apparentée ;
sélectionner une ou plus desdites paires apparentées sur la base des propriétés de ladite molécule de protéine transférée ou de mRNA ; et
sélectionner ladite protéine transférée ou ladite molécule d'acide nucléique désirée en identifiant une molécule sélectionnée à partir du groupe consistant en une molécule mRNA de ladite paire apparentée sélectionnée, une molécule d'acide nucléique complémentaire de ladite molécule mRNA et une molécule d'acide nucléique homologue de ladite molécule mRNA.

**2.** La méthode de la revendication 1 dans laquelle au moins une desdites molécules mRNA candidates et au moins une desdites protéines translatées est liée par une molécule tRNA sélectionnée à partir du groupe consistant en tRNA, tRNA modifié et tRNA analogues.

3. La méthode de la revendication 2 dans laquelle ladite molécule tRNA est connectée à ladite protéine translatée par une peptidyl transférase ribosomale.

4. La méthode de la revendication 2 dans laquelle ladite molécule tRNA est connectée au tRNA par une réticulation induite par ultraviolet entre l'anti-codon de ladite molécule tRNA et le codon du message RNA.

5. La méthode de la revendication 2 dans laquelle ladite molécule tRNA est connectée au mRNA par le thiouracile.

6. La méthode de la revendication 2 dans laquelle ladite molécule tRNA est connectée au mRNA par une réticulation psoralène.

7. La méthode de la revendication 2 dans laquelle ladite molécule tRNA est connectée au mRNA en utilisant un mono adduct de psoralène.

8. La méthode de la revendication 7 dans laquelle ledit mono adduct de psoralène est placé sur ledit mRNA.

9. La méthode de la revendication 7 dans laquelle ledit mono adduct de psoralène est placé sur un anticodon de ladite molécule tRNA.

10. La méthode de la revendication 7 dans laquelle ledit mono adduct de psoralène est placé sur un anticodon d'arrêt de ladite molécule tRNA.

11. La méthode de la revendication 7 dans laquelle ledit mono adduct de psoralène est généré en formant des réticulations et en photo inversant lesdites réticulations en utilisant la lumière visible.

12. La méthode de la revendication 2 dans laquelle ladite molécule tRNA a un accepteur de peptide stable.

13. La méthode de la revendication 2 dans laquelle ladite molécule tRNA est modifiée pour accepter une chaîne peptidique et retenir ladite chaîne d'une manière stable de sorte que la peptidyl transférase ne puisse la détacher.

14. La méthode de la revendication 2 dans laquelle ladite molécule tRNA est modifiée en utilisant un lien qui se lie au ribosome, accepte la chaîne peptidique et ensuite n'agit pas comme un donneur dans la transpeptidation subséquente.

15. La méthode de la revendication 14 dans laquelle ledit lien est sélectionné à partir du groupe consistant en un ester 2' sur une 3'-déoxyadénosine et un amino-acyl tRNA $_{oxred}$.

16. La méthode de la revendication 2 dans laquelle un acide aminé ou un analogue d'acide aminé est attaché à l'extrémité 3' de ladite molécule par un lien stable pour générer un analogue stable d'amino-acyl tRNA.

17. La méthode de la revendication 1 dans laquelle ladite translation s'effectue in vitro.

18. La méthode de la revendication 1 dans laquelle ladite translation s'effectue in situ.

19. La méthode de la revendication 1 dans laquelle ladite paire apparentée est sélectionnée sur la base de liaison de ligand.

20. La méthode de la revendication 1 dans laquelle sélectionner ladite molécule d'acide nucléique désirée comprend :

   avoir une rangée d'acides aminés dans laquelle lesdits acides aminés sont placés dans une position prédéterminée ;
   hybrider au moins une desdites paires apparentées sur ladite rangée ; et
   identifier le mRNA sur la base de sa réaction avec un identificateur de protéine.

21. La méthode de la revendication 20 dans laquelle ladite réaction avec un identificateur de protéine est sélectionnée à partir du groupe consistant en liaison par ligand, immuno-précipitation et réactions enzymatiques.

**22.** Une méthode pour développer une séquence protéique désirée comprenant :

sélectionner au moins une première paire apparentée sur la base d'au moins une caractéristique d'une première protéine translatée ou de ladite molécule mRNA selon la méthode de la revendication 1;

récupérer au moins une desdites premières paires apparentées avec lesdites caractéristiques pour générer au moins une paire apparentée récupérée ;

amplifier un premier composant d'acide nucléique d'une ou plus des dites paires apparentées récupérées;

produire au moins un second composant d'acide nucléique comprenant au moins un desdits premiers composants d'acide nucléique avec une ou plus de variations ;

produire au moins une seconde protéine en translatant au moins un desdits seconds composants d'acide nucléique ;

lier au moins une desdites secondes protéines avec au moins un desdits seconds composants d'acide nucléique pour générer une ou plus de secondes paires apparentées ; et

obtenir la séquence protéique désirée en re-sélectionnant une ou plus desdites secondes paires apparentées sur la base d'au moins une propriété désirées, dans laquelle ladite propriété désirée est la même ou est différente de ladite caractéristique.

**23.** La méthode de la revendication 22 dans laquelle ladite caractéristique désirée est sélectionnée à partir du groupe consistant en propriété de liaison, réactions enzymatiques et modifications chimiques.

# Figure 1

## Formation of Cognate Pair

The codon and the anticodon are connected by UV induced crosslink

The protein and the tRNA are connected by the ribosomal peptidyl transferase.

Ribonucleotide

Anti-codon

tRNA or tRNA Analog modified as in Text

Amino Acid

mRNA

Nascent Protein

# Figure 2

Population of Linked Cognate Pairs

Unselected
Discarded

Repeat

Selection by Protein Characteristic (Especially by Binding Characteristics)

Selected mRNA's Reproduced with Variation (as by Mutation)

Translation with Formation of Cognate Pairs

# Figure 3

Goal Product: Transcript tRNA(Shown Unfolded) With 3'
Puromycin and 5' PO4 and Furan-sided Psoralen Monoadduct
Attaced to the Anticodon Loop

Anticodon Loop ( recognizing
stop codon)

▼

5'PO4 ～～～～～～～～～～～～～～～～～～～～～～～///▤///━━━━━━━━━━━3' Puromycin

▲

Psoralen Monoadduct

Anticodon Loop with
Psoralen Attached

▼

///////▤/////.　　+　　》》》》》》》》》》》》》》》》》》

Bridging DNA

▼

━━━━━━━━━━━━3' Puromycin

Bridging DNA

▼

5'PO4～～～～～～～～～～～～～～～～》》》》》》》》》》》　　+　　////▤////━━━━━━━━━━━3' Puromycin

T4 DNA Ligase ↓

T4 DNA Ligase ↓

5'PO4 ～～～～～～～～～～～～～～～～～～～～///▤///━━━━━━━━━━3' Puromycin

# Figure 4

Psoralen

3'

mRNA

Psoralen furan-sided-monoadducted to either last codon of reading frame or anticodon of SATA.

5'

tRNA or tRNA Analog

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 9831700 A, Szostak
- US 4599303 A
- US 5462733 A
- EP 01935701 A
- US 0116210 W
- WO 09859809 A
- WO 60206016 A

### Non-patent literature cited in the description

- **TUERK ; GOLD.** *Science,* 1990, vol. 249, 505
- **FIELDS ; SONG.** *Nature,* 1989, vol. 340, 245
- **LICITRA ; LIU.** *PNAS,* 1996, vol. 93, 12817
- **BODER ; WITTRUP.** *Nat Biotechnol,* 1997, vol. 15, 553
- **SCOTT ; SMITH.** *Science,* 1990, vol. 249, 386
- **MATTHEAKIS et al.** *PNAS,* 1994, vol. 91, 9022
- **HANES ; PLÜCKTHUN.** *PNAS,* 1997, vol. 94, 4937
- **JERMUTUS et al.** *Current Opinion in Biotechnology,* 1998, vol. 9, 534
- **ROBERTS ; SZOSTAK.** *PNAS,* 1997, vol. 94, 12297
- **NEMOTO et al.** *FEBS Letters,* 1997, vol. 414, 405
- **ROBERTS.** *Curr. Opin. Chem. Biol.,* 1999, vol. 3, 268
- **CHINALI et al.** *Biochem.,* 1974, vol. 13, 3001
- **KRAYEVSKY ; KUKHANOVA.** *Prog. Nuc. Acid Res,* 1979, vol. 23, 1
- **SPRINZL ; CRAMER.** *Prog. Nuc. Acid Res,* 1979, vol. 22, 1
- **FRASER ; RICH.** *PNAS,* 1973, vol. 70, 2671
- **CIMINO et al.** *Ann. Rev. Biochem.,* 1985, vol. 54, 1151
- **GELLER ; RICH.** *Nature,* 1980, vol. 283, 41
- **EDWARDS et al.** *PNAS,* 1991, vol. 88, 1153
- **HOU ; SCHIMMEL.** *Biochem,* 1989, vol. 28, 6800
- **LUCAS-LENARD ; HAENNI.** *PNAS,* 1969, vol. 63, 93
- **DABROWSKI et al.** *EMBO J.,* 1995, vol. 14, 4872
- **HARRINGTON et al.** *Biochem.,* 1993, vol. 32, 7617
- **MOORE ; SHARP.** *Science,* 1992, vol. 256, 992
- **ROMANIUK ; UHLENBECK.** *Methods in Enzymology,* 1983, vol. 100, 52
- **SPEILMANN et al.** *PNAS,* 1992, vol. 89, 4514
- **GAMPER et al.** *J. Mol. Biol.,* 1987, vol. 197, 349
- **GAMPER et al.** *Photochem. Photobiol.,* 1984, vol. 40, 29
- **GASPARRO et al.** *Photochem. Photobiol.,* 1993, vol. 57, 1007
- **SPRINZL ; CRAMER.** *Prog. Nuc. Acid Res.,* 1979, vol. 22, 1
- **VOET ; VOET.** Biochemistry. John Wiley and Sons, 1995, 1000-1002
- **VOET ; VOET.** Biochemistry. John Wiley and Sons, 1995, 380
- **CIMINO et al.** *Biochem,* 1986, vol. 25, 3013
- **THOMPSON J. F et al.** *Biochemistry,* vol. 21, 1363
- **SASTRY et al.** *J. Photochem. Photobiol. B Biol.,* vol. 14, 65-79
- **SPIELMANN et al.** *PNAS,* vol. 89, 4514-4518
- **ROMANIUK ; UHLENBECK.** *Methods in Enzymology,* 1983, vol. 100, 52-59
- **UHLENBECK.** *Biochemistry,* 1985, vol. 24, 2705-2712
- **SAMBROOK.** Molecular Cloning, A Laboratory Manual. Coldspring Harbor Press, 1989

SEQUENCE LISTING

<110> Williams, Richard B.

<120> IN VITRO EVOLUTION OF NUCLEIC ACIDS AND ENCODED POLYPEPTIDE

<130> PRONOV.001VEP

<140> EP 01 935 701.1
<141> 2001-05-18

<150> PCT/US01/16210
<151> 2001-05-18

<150> 09/859,809
<151> 2001-05-17

<150> 60/206,016
<151> 2000-05-19

<160> 7

<170> FastSEQ for Windows Version 4.0

<210> 1
<211> 13
<212> RNA
<213> Artificial Sequence

<220>
<223> Precursor RNA for formation of psoralentated RNA fragment

<221> modified_base
<222> (6)...(6)
<223> n=p

<400> 1
cuagancugg agg          13


<210> 2
<211> 13
<212> RNA
<213> Artificial Sequence


<220>
<223> Furan sided psoralentated RNA fragment


<221> misc_feature
<222> (2)...(3)
<223> psoralen bound to UA


<221> modified_base
<222> (6)...(6)
<223> n=p


<400> 2
cuagancugg agg          13


<210> 3
<211> 23
<212> RNA
<213> Artificial Sequence


<220>
<223> Precursor RNA for psoralentated RNA fragment


<221> misc_feature
<222> (1)...(5)
<223> n=g, a, u, or c


<221> misc_feature
<222> (19)...(23)
<223> n=g, a, u, or c


<400> 3
nnnnnccucc agaucuagnn nnn          23


<210> 4
<211> 23
<212> RNA
<213> Artificial Sequence


<220>
<223> Psoralentated RNA fragment


<221> misc_feature
<222> (16)...(17)
<223> psoralen bound to UA


<221> misc_feature
<222> (1)...(5)
<223> n=g, a, u, or c


<221> misc_feature

<222> (19)...(23)
<223> n=g, a, u, or c

<400> 4
nnnnnccucc agaucuagnn nnn          23

<210> 5
<211> 30
<212> DNA
<213> Artificial Sequence

<220>
<223> Fragment 3; modified tRNA; thymine at residue 8 before pseudouridine

<221> modified_base
<222> (9)...(9)

<223> n=p

<221> misc feature
<222> (30)...(30)
<223> n=puromycin

<400> 5
uccugugtnc gauccacaga auucgcaccn          30

<210> 6
<211> 43
<212> DNA
<213> Artificial Sequence

<220>
<223> Fragment 2+3; modified tRNA; thymine at residue 21 before pseudouridine

<221> modified_base
<222> (22)...(22)
<223> n=p

<221> misc_feature
<222> (43)...(43)
<223> n=puromycin

<221> modified_base
<222> (6)...(6)
<223> n=p

<400> 6
cuagancugg agguccugug tncgauccac agaauucgca ccn          43

<210> 7
<211> 76
<212> DNA
<213> Artificial Sequence

<220>
<223> Fragment 1 + 2 + 3; modified tRNA; thymine at residue 54 before pseudouridine

<221> modified base

```
<222> (39)...(39)
<223> n=p

<221> modified_base
<222> (55)...(55)
<223> n=p

<221> misc_feature
<222> (76)...(76)
<223> n=puromycin

<400> 7


gcggauuuag cucagddggg agagcgccag acucuaganc uggagguccu gugtncgauc 60
cacagaauuc gcaccn                                                 76
```